# FASCICULE DE BREVET EUROPEEN

(11) **EP 2 890 331 B1**
(45) Date de publication et mention de la délivrance du brevet: **21.09.2016**
(21) Numéro de dépôt: 13773335.8
(22) Date de dépôt: 08.08.2013
(51) Int. Cl.: A61F 2/42, A61F 2/08, A61F 2/46

(54) **PROTHÈSE POUR LA RESTAURATION D'UNE ARTICULATION ENTRE DEUX OS ET ANCILLAIRE POUR SON ASSEMBLAGE**
PROTHESE ZUR WIEDERHERSTELLUNG EINES GELENKES ZWISCHEN ZWEI KNOCHEN UND HILFSWERKZEUG ZUR MONTAGE DAFÜR
PROSTHESIS FOR THE RESTORATION OF A JOINT BETWEEN TWO BONES, AND ANCILLARY TOOL FOR THE ASSEMBLY THEREOF

(30) Priorité: 29.08.2012 FR 1202321
(43) Date de publication de la demande: 08.07.2015
(73) Titulaire: Biover, 6052 Hergiswil (CH)
(72) Inventeur: MERLE, Michel, L-1912 Luxembourg (LU); LEU, Beat, CH-6374 Buochs (CH)
(74) Mandataire: Flavenot, Bernard
(86) Numéro de dépôt international: PCT/IB2013/001757
(87) Numéro de publication internationale: WO 2014/033516

(56) Documents cités:
- FR-A1- 2 634 373
- US-A- 3 946 446
- US-A- 5 534 033
- US-A1- 2010 191 342

## Description

La présente invention concerne les prothèses pour la restauration d'une articulation entre deux os, qui trouvent une application particulièrement avantageuse, mais non exclusivement, comme prothèses articulaires métacarpo-phalangiennes et inter phalangiennes proximales.

La présente invention concerne aussi un ancillaire pour l'aide à l'assemblage d'une telle prothèse.

Il existe des prothèses articulaires, notamment pour le remplacement des articulations métacarpo-phalangiennes et inter phalangiennes proximales. De telles prothèses sont par exemple décrites et illustrées dans les US 2010/191342, US 3 946 446, US 5 534 033 et FR 2 634 373.

Ces prothèses connues donnent de bons résultats, mais présentent des inconvénients essentiellement dus à la complexité de leur structure et donc de leur assemblage, et au fait que leur structure dépend du mode d'implantation utilisé par le praticien.

La présente invention a ainsi pour but de réaliser une prothèse pour la restauration d'une articulation entre deux os, qui trouvent une application particulièrement avantageuse, mais non exclusivement, comme prothèse articulaire métacarpo-phalangienne et inter phalangienne proximale, qui soit d'une structure très simple, facile à assembler et à implanter, et qui en outre puisse être implantée selon de nombreuses voies chirurgicales en accord avec les habitudes et souhaits des praticiens qui opèrent, essentiellement par les trois voies dorsale, latérale ou palmaire qui sont les plus couramment adoptées par les Chirurgiens.

Plus précisément, la présente invention a pour objet une prothèse pour la restauration d'une articulation entre des premier et second os, comportant :
- une première platine,
- des moyens pour associer cette première platine avec une extrémité réséquée du premier os,
- une seconde platine,
- des moyens pour associer cette seconde platine avec une extrémité réséquée du second os, et
- des moyens pour monter les deux platines en rotation l'une par rapport à l'autre autour d'un axe de rotation,
caractérisée par le fait que les moyens pour monter les deux platines en rotation l'une par rapport à l'autre autour de l'axe de rotation sont constitués par:
- une charnière flexible, ladite charnière flexible comportant :
   * une lamelle flexible plane affectant une forme sensiblement cylindrique,
   * deux renflements oblongs affectant une forme sensiblement cylindrique, et
   * des moyens pour associer ces deux renflements oblongs respectivement à deux bords opposés de ladite lamelle flexible de façon que les génératrices des formes cylindriques de la lamelle et des deux renflements soient sensiblement parallèles au dit axe de rotation, la dimension de la section transversale respectivement de ces deux renflements, prise suivant une direction perpendiculaire au plan contenant ledit axe de rotation et traversant les deux renflements, étant supérieure à l'épaisseur de ladite lamelle flexible prise suivant la même direction,
- une percée réalisée dans chacune des deux platines, chaque percée comportant une extrémité débouchant sur une première face de la platine dans laquelle elle est réalisée, les deux percées ayant respectivement une forme complémentaire respectivement des deux renflements,
- une saignée réalisée dans chacune des deux platines, la saignée réalisée dans une platine débouchant dans la percée réalisée dans cette platine et sur une seconde des faces de ladite platine, et comportant une extrémité débouchant sur la première face de la platine,
- la charnière flexible et les deux platines étant montées en coopération de façon que les deux renflements et la lamelle flexible soient respectivement situés dans les deux percées et dans les deux saignées.

La présente invention a aussi pour objet un ancillaire pour l'aide à l'assemblage de la prothèse définie ci-dessus caractérisé par le fait qu'il comporte :
- un guide dont une extrémité comporte une empreinte en creux d'au moins une partie des deux platines quand elles sont dans la position dans laquelle elles sont aptes à être associées par la charnière flexible,
- une traversée réalisée de part en part dans ledit guide et ayant une section transversale complémentaire de celle de ladite charnière flexible, ladite traversée étant réalisée dans ledit guide de façon à être parfaitement en regard des dites extrémités des percées et des saignées situées sur la première face des deux platines quand les deux platines sont enfichées dans leur empreinte en creux respective, et
- un poussoir pour translater, par poussée, ladite charnière flexible dans ladite traversée pour positionner cette dite charnière flexible en coopération avec les deux platines quand les deux platines sont enfichées dans leur empreinte en creux respective, de façon que les deux renflements et la lamelle flexible viennent respectivement se loger dans les deux percées et dans les deux saignées.

D'autres caractéristiques et avantages de la présente invention apparaîtront au cours de la description suivante donnée en regard des dessins annexés à titre illustratif mais nullement limitatif, dans lesquels :
La figure 1 est une vue en coupe et de côté d'un mode de réalisation de la prothèse selon l'invention pour la restauration d'une articulation entre deux os,
La figure 2 est une vue en perspective et en éclaté de la prothèse selon l'invention en accord avec la représentation de la figure 1, en coopération avec un mode de réalisation de l'ancillaire selon l'invention pour l'aide à son assemblage, et
La figure 3 est une vue en coupe et de côté d'une partie d'un autre mode Il est bien précisé que, dans la présente description, si l'adverbe "sensiblement" est associé à un qualificatif d'un moyen donné, ce qualificatif doit être compris au sens strict ou approché.

La présente invention est relative à une prothèse pour la restauration d'une articulation entre des premier et second os 01, O2, par exemple une articulation métacarpo-phalangienne et inter phalangienne proximale.

En référence aux trois figures annexées, la prothèse comporte une première platine 11, des moyens 12 pour associer cette première platine 11 avec une extrémité réséquée du premier os 01, une seconde platine 21, des moyens 22 pour associer cette seconde platine 21 avec une extrémité réséquée du second os 02, et des moyens 30 pour monter les deux platines 11, 21 en rotation l'une par rapport à l'autre autour d'un axe de rotation 31.

Selon une caractéristique de l'invention, les moyens 30 pour monter les deux platines 11, 21 en rotation l'une par rapport à l'autre autour de l'axe de rotation 31 sont constitués par une charnière flexible 40 comportant une lamelle flexible plane 41 affectant une forme sensiblement cylindrique, deux renflements oblongs 42, 43 affectant une forme sensiblement cylindrique, et des moyens pour associer ces deux renflements oblongs 42, 43 respectivement à deux bords opposés de la lamelle flexible 41 de façon que les génératrices des formes cylindriques de la lamelle et des deux renflements soient sensiblement parallèles à l'axe de rotation 31, la dimension de la section transversale respectivement de ces deux renflements 42, 43, prise suivant une direction perpendiculaire au plan contenant ledit axe de rotation 31 et traversant les deux renflements, étant supérieure à l'épaisseur de ladite lamelle flexible 41 prise suivant la même direction.

Il est précisé que les formes des deux renflements oblongs 42, 43 illustrées sur les figures ne sont données qu'à titre illustratif et que, bien entendu, ces renflements oblongs peuvent affecter une forme sensiblement cylindrique de révolution ou non, de toute section transversale, par exemple circulaire, polygonale, etc.

Avantageusement les deux renflements 42, 43 et la lamelle 41 sont réalisés d'une seule pièce par exemple en polycarbonate de préférence thermoplastique ou analogue, avec une épaisseur de la lamelle déterminée de façon qu'elle soit à la fois relativement souple et élastique, et résistante pour ne pas casser.

La prothèse comporte en outre une percée 44, 45 réalisée dans chacune des deux platines 11, 21, chaque percée comportant une extrémité débouchant sur une première face de la platine dans laquelle elle est réalisée, les deux percées 44, 45 ayant respectivement une forme complémentaire respectivement des deux renflements 42, 43. De préférence, la percée débouchera à ses deux extrémités sur deux premières faces opposées de la platine.

Elle comporte aussi une saignée 46, 47 réalisée dans chacune des deux platines 11, 21, la saignée réalisée dans une platine débouchant dans la percée réalisée dans cette platine et sur une seconde des faces de la platine, et comportant une extrémité débouchant sur la première face de la platine. De préférence, la saignée débouchera à ses deux extrémités sur les deux premières faces opposées de la platine.

De plus la charnière flexible 40 et les deux platines 11, 21 sont montées en coopération de façon que les deux renflements 42, 43 et la lamelle flexible 41 soient respectivement situés dans les deux percées 44, 45 et dans les deux saignées 46, 47.

De façon possible, la prothèse comporte au moins un renfort noyé dans la matière dans laquelle est réalisée la charnière flexible (40), comme des axes métalliques ou analogues, pour renforcer par exemple les deux renflements 42, 43.

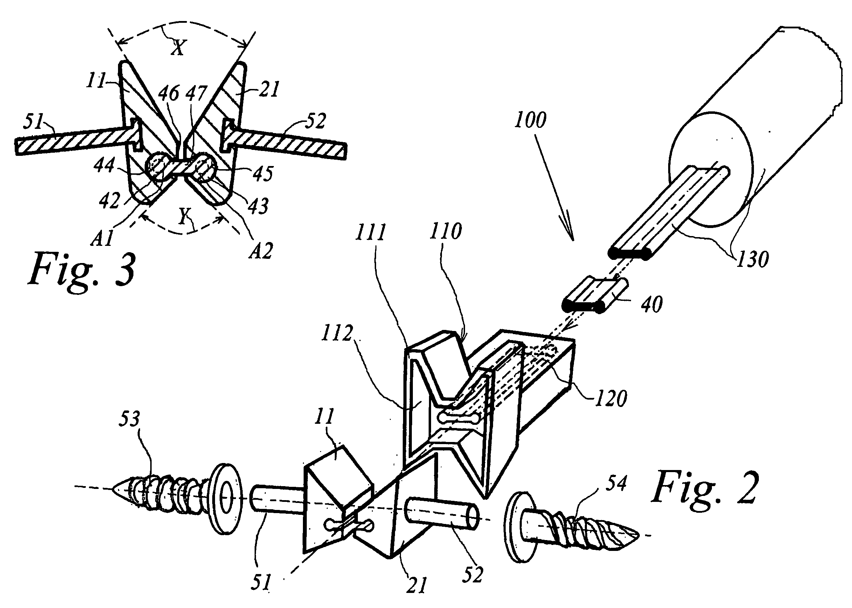

A titre d'exemple préférentiel, le mode de réalisation représenté sur la figure 3 évoque cette possibilité, la prothèse comportant deux âmes A1, A2 illustrées en traits interrompus, qui sont respectivement noyées sensiblement au centre des renflements 42, 43, pour assurer un maintien efficace et durable de ces renflements dans les percées 44, 45.

De même, il est précisé que cette charnière 40 peut aussi être réalisée au moyen de plusieurs matériaux flexibles, soit en couches les unes sur les autres, soit en mélange de matériaux de différentes flexibilités. L'homme du métier saura, en fonction des résultats à obtenir, réaliser la charnière flexible 40 qui convient.

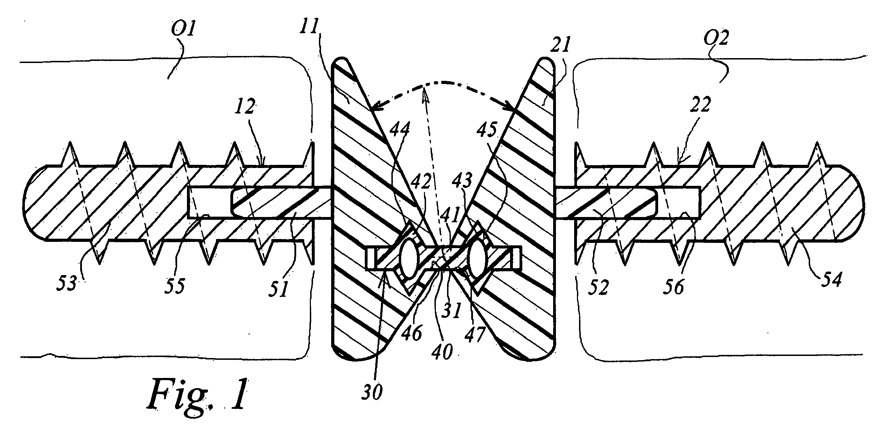

Selon une autre caractéristique très préférentielle de l'invention, chaque platine est de forme cylindrique, sa section transversale constituant la directrice de la forme cylindrique, définie perpendiculairement aux génératrices, ayant la forme sensiblement d'un triangle dont l'un des sommets a une valeur d'angle supérieure à quatre-vingt-dix degrés, très avantageusement une valeur d'angle sensiblement égale à cent-deux degrés. Les deux triangles des deux platines sont définis, dans ces conditions, de façon que, quand la charnière flexible n'est pas soumise à un effort de torsion, donc au repose comme illustré sur les figures 1 et 3, les valeurs des deux angles X et Y aient des valeurs sensiblement égales respectivement à soixante-cinq degrés et quatre-vingt-dix degrés.

De façon encore plus préférentielle, la saignée 46, 47 réalisée dans chaque platine 11, 21 débouche sensiblement sur le un des sommets du triangle défini ci-dessus et est réalisée suivant la hauteur du triangle passant par ce un sommet.

De façon très avantageuse, pour des facilités de fabrication, de réduction de coût et de gestion à tous les niveaux, les deux platines 11, 21 sont sensiblement identiques, comme illustré sur les trois figures, et sont réalisées dans divers matériaux, mais de façon avantageuse par thermoformage avec du polycarbonate thermoplastique ou analogue, comme la charnière flexible 40.

Selon une réalisation préférée, les moyens 12, 22 pour associer une des platines 11, 21 avec une extrémité réséquée d'un des deux os 01, 02 sont constitués par une patte 51, 52, des moyens pour solidariser la patte avec la platine, une vis osseuse 53, 54, et des moyens pour associer la patte avec la vis osseuse.

Les deux vis osseuses sont de préférence en titane et auto-taraudeuses, ce qui permet leur ostéo-intégration dans la cavité médullaire des os, par exemple, selon une application particulièrement avantageuse, des phalanges et des métacarpiens.

Selon une réalisation possible et préférée, les moyens pour associer la patte avec la vis osseuse sont constitués par le fait que la patte 51, 52 est de forme cylindrique et que, dans la vis osseuse, est réalisé un logement borgne 55, 56 de forme cylindrique, la section transversale de ce logement étant sensiblement complémentaire de celle de la patte de façon que cette dernière soit apte à y coulisser en translation.

En outre, de façon tout à fait préférentielle, la patte 51, 52 est de forme cylindrique de révolution de façon qu'elle soit apte à pivoter dans le logement borgne 55, 56 autour de son axe longitudinal. Très préférentiellement, les deux pattes sont toutes les deux de forme cylindrique de révolution et identiques.

Chaque platine, au moyen de la patte cylindrique de révolution, vient s'ajuster dans sa vis osseuse correspondante, ce qui permet un effet piston en translation, mais aussi si nécessaire de rotation pour un ajustement en position de la platine sur la face réséquée de l'os, car la prothèse doit travailler aussi bien en distraction qu'en compression.

Quant aux moyens pour solidariser la patte avec la platine, ils peuvent être de différentes formes, par exemple constitués par l'une des réalisations suivantes : réalisation de la patte et de la platine d'une seule pièce comme illustré sur la figure 1, ou montage amovible de la patte avec la platine, par exemple un montage en queue d'aronde comme illustré sur la figure 3, ou clipsage, ou analogue.

Le mode d'implantation d'une telle prothèse est du domaine des praticiens et ne sera pas spécifiquement développé ici, d'autant plus qu'il n'entre pas dans le champ de protection de l'invention.

La présente invention a aussi pour objet, figure 2, un ancillaire 100 pour l'aide à l'assemblage d'une prothèse telle que définie ci-dessus.

Cet ancillaire comporte un guide 110 dont une extrémité 111 comporte un plateau apte à prendre appui sur les deux platines, ce plateau comprenant avantageusement une empreinte en creux 112 d'au moins une partie des deux platines 11, 21 quand elles sont dans la position dans laquelle elles sont aptes à être associées par la charnière flexible 40, une traversée 120 réalisée de part en part dans ce guide 110 et ayant une section transversale complémentaire de celle de la charnière flexible 40, la traversée étant réalisée dans le guide 110 de façon à être parfaitement en regard des extrémités des percées 44, 45 et des saignées 46, 47 situées sur la première face des deux platines 11, 21 quand les deux platines sont enfichées dans leur empreinte en creux respective, et un poussoir 130 pour translater, par poussée, la charnière flexible 40 dans la traversée 120 pour positionner cette charnière flexible 40 en coopération avec les deux platines 11, 21 quand les deux platines sont enfichées dans leur empreinte en creux respective, de façon que les deux renflements 42, 43 et la lamelle flexible 41 viennent respectivement se loger dans les deux percées 44, 45 et dans les deux saignées 46, 47.

L'utilisation et le fonctionnement d'un tel ancillaire se déduit sans aucune difficulté de la description faite ci-dessus. Ils ne seront donc pas plus amplement décrits ici, dans l'unique souci de simplifier la présente description.

## Revendications

1. Prothèse pour la restauration d'une articulation entre des premier et second os (01, 02), comportant :
• une première platine (11),
• des moyens (12) pour associer cette première platine (11) avec une extrémité réséquée du premier os (01),
• une seconde platine (21),
• des moyens (22) pour associer cette seconde platine (21) avec une extrémité réséquée du second os (02), et
• des moyens (30) pour monter les deux platines (11, 21) en rotation l'une par rapport à l'autre autour d'un axe de rotation (31), les moyens (30) pour monter les deux platines (11, 21) en rotation l'une par rapport à l'autre autour de l'axe de rotation (31) étant constitués par :
• une charnière flexible (40), ladite charnière flexible comportant :
* une lamelle flexible plane (41) affectant une forme sensiblement cylindrique, et
* deux renflements oblongs (42, 43) affectant une forme sensiblement cylindrique,
la dimension de la section transversale respectivement de ces deux renflements (42, 43), prise suivant une direction perpendiculaire au plan contenant ledit axe de rotation (31) et traversant les deux renflements, étant supérieure à l'épaisseur de ladite lamelle flexible (41) prise suivant la même direction,
• une percée (44, 45) réalisée dans chacune des deux platines (11, 21), chaque percée comportant une extrémité débouchant sur une première face de la platine dans laquelle elle est réalisée, les deux percées (44, 45) ayant respectivement une forme complémentaire respectivement des deux renflements (42, 43),
• une saignée (46, 47) réalisée dans chacune des deux platines (11, 21), la saignée réalisée dans une platine débouchant dans la percée réalisée dans cette platine et sur une seconde de ses faces, et comportant une extrémité débouchant sur la première face de la platine,
• la charnière flexible (40) et les deux platines (11, 21) étant montées en coopération de façon que les deux renflements (42, 43) et la lamelle flexible (41) soient respectivement situés dans les deux percées (44, 45) et dans les deux saignées (46, 47), **caractérisée en ce que** la charnière flexible comporte des moyens pour associer ces deux renflements oblongs (42, 43) respectivement à deux bords opposés de ladite lamelle flexible (41) de façon que les génératrices des formes cylindriques de la lamelle et des deux renflements soient sensiblement parallèles au dit axe de rotation (31).

2. Prothèse selon la revendication 1, **caractérisée par le fait que** chaque platine est de forme cylindrique, sa section transversale constituant la directrice de la forme cylindrique, définie perpendiculairement aux génératrices, ayant la forme sensiblement d'un triangle dont l'un des sommets a une valeur d'angle supérieure à quatre-vingt-dix degrés.

3. Prothèse selon la revendication 2, **caractérisée par le fait que** la saignée (46, 47) réalisée dans chaque platine (11, 21) débouche sensiblement sur ledit un des sommets du triangle et est réalisée suivant la hauteur du triangle passant par ledit un sommet.

4. Prothèse selon la revendication 3, **caractérisée par le fait que** les deux platines (11, 21) sont sensiblement identiques.

5. Prothèse selon l'une des revendications précédentes, **caractérisée par le fait qu'**elle comporte au moins un renfort (A1, A2) noyé dans la matière dans laquelle est réalisée ladite charnière flexible (40).

6. Prothèse selon l'une des revendications précédentes, **caractérisée par le fait que** les moyens (12, 22) pour associer une des platines (11, 21) avec une extrémité réséquée d'un des deux os (01, 02) sont constitués par :
• une patte (51, 52),
• des moyens pour solidariser la patte avec la platine,
• une vis osseuse (53, 54) optionnellement auto-taraudeuse, et
• des moyens pour associer ladite patte avec ladite vis osseuse.

7. Prothèse selon la revendication 6, **caractérisée par le fait que** les moyens pour associer ladite patte avec ladite vis osseuse sont constitués par **le fait que** la patte (51, 52) est de forme cylindrique et que, dans la vis osseuse, est réalisé un logement borgne (55, 56) de forme cylindrique, la section transversale de ce dit logement étant sensiblement complémentaire de celle de ladite patte de façon que cette dernière soit apte à coulisser en translation dans ledit logement borgne.

8. Prothèse selon la revendication 7, **caractérisée par le fait que** la patte (51, 52) est de forme cylindrique de révolution de façon qu'elle soit apte à pivoter dans ledit logement borgne (55, 56) autour de son axe longitudinal, optionnellement les deux pattes (51, 52) sont identiques et de forme cylindrique de révolution.

9. Prothèse selon l'une des revendications 6 à 8, **caractérisée par le fait que** les moyens pour solidariser la patte avec la platine sont constitués par l'une des réalisations suivantes : réalisation de la patte et de la platine d'une seule pièce, montage amovible de ladite patte avec ladite platine.

10. Prothèse selon l'une des revendications précédentes quand elle dépend de la revendication 2, **caractérisée par le fait que** ledit un sommet a une valeur d'angle sensiblement égale à cent-deux degrés.

11. Prothèse selon l'une des revendications 1 à 9 en combinaison avec un ancillaire (100) pour l'aide à l'assemblage de ladite prothèse, , **caractérisé par le fait que** l'ancillaire comporte :
• un guide (110),
• une traversée (120) réalisée de part en part dans ledit guide (110) et ayant une section transversale complémentaire de celle de ladite charnière flexible (40), ladite traversée étant réalisée dans ledit guide (110) de façon à être parfaitement en regard des dites extrémités des percées (44, 45) et des saignées (46, 47) situées sur la première face des deux platines (11, 21), et
• un poussoir (130) pour translater, par poussée, ladite charnière flexible (40) dans ladite traversée (120) pour positionner cette dite charnière flexible (40) en coopération avec les deux platines (11, 21) de façon que les deux renflements (42, 43) et la lamelle flexible (41) viennent respectivement se loger dans les deux percées (44, 45) et dans les deux saignées (46, 47).

12. Prothèse selon l'une des revendications 1 à 9 en combinaison avec un ancillaire (100) selon la revendication 11, **caractérisé par le fait que** ledit guide (110) est constitué d'un plateau qui comporte, à une extrémité (111), une empreinte en creux (112) d'au moins une partie des deux platines (11, 21) quand elles sont dans la position dans laquelle elles sont aptes à être associées par la charnière flexible (40).

## Patentansprüche

1. Prothese für die Wiederherstellung eines Gelenks zwischen einem ersten und einem zweiten Knochen (01, 02), die umfasst:
• eine erste Platine (11),
• Mittel (12), um diese erste Platine (11) mit einem resezierten Ende des ersten Knochens (O1) zu verbinden,
• eine zweite Platine (21),
• Mittel (22), um diese zweite Platine (21) mit einem resezierten Ende des zweiten Knochens (02) zu verbinden, und
• Mittel (30), um die zwei Platinen (11, 21) relativ zueinander um eine Drehachse (31) drehbar zu montieren,
wobei die Mittel (30), um die zwei Platinen (11, 21) relativ zueinander um die Drehachse (31) drehbar zu montieren, gebildet sind durch:
• ein flexibles Scharnier (40), wobei das flexible Scharnier Folgendes umfasst:
* eine ebene flexible Lamelle (41), die eine im Wesentlichen zylindrische Form aufweist, und
* zwei lang gestreckte Verdickungen (42, 43), die eine im Wesentlichen zylindrische Form aufweisen, wobei die Abmessung des jeweiligen Querschnitts dieser zwei Verdickungen (42, 43) in einer Richtung senkrecht zu der Ebene, die die Drehachse (31) enthält und durch die zwei Verdickungen verläuft, größer als die Dicke der flexiblen Lamelle (41) in derselben Richtung ist,
• eine Durchlochung (44, 45), die in jeder der zwei Platinen (11, 21) verwirklicht ist, wobei jede Durchlochung ein Ende aufweist, das in eine erste Fläche der Platine, in der sie verwirklicht ist, mündet, wobei die zwei Durchlochungen (44, 45) jeweils eine zu den zwei Verdickungen (42, 43) komplementäre Form haben,
• einen Schlitz (46, 47), der in jeder der zwei Platinen (11, 21) verwirklicht ist, wobei der Schlitz, der in einer Platine verwirklicht ist, in die in dieser Platine verwirklichte Durchlochung und in eine zweite ihrer Flächen mündet und ein Ende aufweist, das in die erste Fläche der Platine mündet,
• wobei das flexible Scharnier (40) und die zwei Platinen (11, 21) zusammenwirkend montiert sind, derart, dass die zwei Verdickungen (42, 43) und die flexible Lamelle (41) sich entsprechend in den zwei Durchlochungen (44, 45) und in den zwei Schlitzen (46, 47) befinden, **dadurch gekennzeichnet, dass** das flexible Scharnier Mittel umfasst, um diese zwei lang gestreckten Verdickungen (42, 43) entsprechend mit zwei gegenüberliegenden Rändern der flexiblen Lamelle (41) zu verbinden, derart, dass die Erzeugenden der zylindrischen Formen der Lamelle und der zwei Verdickungen zu der Drehachse (31) im Wesentlichen parallel sind.

2. Prothese nach Anspruch 1, **dadurch gekennzeichnet, dass** jede Platine eine zylindrische Form hat, deren Querschnitt die Leitlinie der zylindrischen Form bildet, senkrecht zu den Erzeugenden definiert ist und im Wesentlichen die Form eines Dreiecks hat, wovon einer der Scheitel einen Winkelwert besitzt, der größer als neunzig Grad ist.

3. Prothese nach Anspruch 2, **dadurch gekennzeichnet, dass** der Schlitz (46, 47), der in jeder Platine (11, 21) verwirklicht ist, im Wesentlichen in einen der Scheitelpunkte des Dreiecks mündet und längs der Höhe des Dreiecks, die durch diesen Scheitelpunkt geht, verwirklicht ist.

4. Prothese nach Anspruch 3, **dadurch gekennzeichnet, dass** die zwei Platinen (11, 21) im Wesentlichen gleich sind.

5. Prothese nach einem der vorhergehenden Ansprüche, **dadurch gekennzeichnet, dass** sie wenigstens eine Verstärkung (A1, A2) umfasst, die in das Material eingelassen ist, in dem das flexible Scharnier (40) verwirklicht ist.

6. Prothese nach einem der vorhergehenden Ansprüche, **dadurch gekennzeichnet, dass** die Mittel (12, 22) zum Verbinden einer der Platinen (11, 21) mit einem resezierten Ende eines der zwei Knochen (01, 02) gebildet sind durch:
• einen Haken (51, 52),
• Mittel, um den Haken an der Platine zu befestigen,
• eine Knochenschraube (53, 54), die optional selbstschneidend ist, und
• Mittel, um den Haken mit der Knochenschraube zu verbinden.

7. Prothese nach Anspruch 6, **dadurch gekennzeichnet, dass** die Mittel, um den Haken mit der Knochenschraube zu verbinden, dadurch gebildet sind, dass der Haken (51, 52) eine zylindrische Form hat und dass in der Knochenschraube ein Blindaufnahmeraum (55, 56) mit zylindrischer Form verwirklicht ist, wobei der Querschnitt dieses Aufnahmeraums zu jenem des Hakens im Wesentlichen komplementär ist, derart, dass dieser Letztere in dem Blindaufnahmeraum in Längsrichtung gleiten kann.

8. Prothese nach Anspruch 7, **dadurch gekennzeichnet, dass** der Haken (51, 52) eine rotationssymmetrische zylindrische Form hat, derart, dass er sich in dem Blindaufnahmeraum (55, 56) um seine Längsachse drehen kann, wobei die zwei Haken (51, 52) optional gleich sind und eine rotationssymmetrische zylindrische Form haben.

9. Prothese nach einem der Ansprüche 6 bis 8, **dadurch gekennzeichnet, dass** die Mittel, um den Haken an der Platine zu befestigen, durch eine der folgenden Ausbildungen gebildet sind: einteilige Ausbildung des Hakens und der Platine, lösbare Montage des Hakens an der Platine.

10. Prothese nach einem der vorhergehenden Ansprüche, wenn abhängig von Anspruch 2, **dadurch gekennzeichnet, dass** einer der Scheitelpunkte einen Winkelwert besitzt, der im Wesentlichen gleich hundertzwei Grad ist.

11. Prothese nach einem der Ansprüche 1 bis 9 in Kombination mit einem Hilfselement (100) für die Unterstützung bei der Anbringung der Prothese, **dadurch gekennzeichnet, dass** das Hilfselement Folgendes umfasst:
• eine Führung (110),
• eine Durchführung (120), die beiderseits der Führung (110) verwirklicht ist und einen Querschnitt besitzt, der zu jenem des flexiblen Scharniers (40) komplementär ist, wobei die Durchführung in der Führung (110) in der Weise verwirklicht ist, dass sie sich genau gegenüber den beiden Enden der Durchlochungen (44, 45) und der Schlitze (46, 47) befinden, die in der ersten Fläche der zwei Platinen (11, 21) vorhanden sind, und
• einen Drücker (130), um das flexible Scharnier (40) in der Durchführung (120) durch Schub in Längsrichtung zu bewegen, um dieses flexible Scharnier (40) in Zusammenwirkung mit den zwei Platinen (11, 21) in der Weise zu positionieren, dass die zwei Verdickungen (42, 43) und die flexible Lamelle (41) entsprechend in den beiden Durchlochungen (44, 45) bzw. in den zwei Schlitzen (46, 47) aufgenommen werden.

12. Prothese nach einem der Ansprüche 1 bis 9 in Kombination mit einem Hilfselement (100) nach Anspruch 11, **dadurch gekennzeichnet, dass** die Führung (110) aus einer Platte gebildet ist, die an einem Ende (111) einen vertieften Eindruck (112) wenigstens eines Teils der zwei Platinen (11, 21) aufweist, wenn diese in der Position sind, in der sie durch das flexible Scharnier (40) verbunden werden können.

## Claims

1. A prosthesis for restoring a joint between first and second bones (01, 02), the prosthesis comprising:
• a first plate (11);
• means (12) for associating this first plate (11) with a resected end of the first bone (01);
• a second plate (21);
• means (22) for associating this second plate (21) with a resected end of the second bone (02); and
• means (30) for mounting the two plates (11, 21) pivotally relative to each other about a pivot axis (31);the means (30) for mounting the two plates (11, 21) pivotally relative to each other about the pivot axis (31) being constituted by:
• a flexible hinge (40), said flexible hinge comprising:
* a plane flexible strip (41) of substantially cylindrical shape; and
* two oblong swellings (42, 43) of substantially cylindrical shape, the dimension of the cross-section of each of these two swellings (42, 43) respectively, taken in a direction perpendicular to the plane containing said pivot axis (31) and passing through the two swellings, being greater than the thickness of said flexible strip (41) taken in the same direction;
• a hole (44, 45) made in each of the two plates (11, 21), each hole having an end opening out in a first face of the plate in which it is made, the two holes (44, 45) having respective shapes complementary to the two respective swellings (42, 43);
• a slot (46, 47) made in each of the two plates (11, 21), the slot made in a plate opening out into the hole made in that plate and into a second one of its faces, and including an end opening out into the first face of the plate; and
• the flexible hinge (40) and the two plates (11, 21) being mounted in cooperation in such a manner that the two swellings (42, 43) and the flexible strip (41) are situated respectively in the two holes (44, 45) and in the two slots (46, 47), **characterized by** the fact that the flexible hinge comprises means for associating these two oblong swellings (42, 43) respectively with two opposite edges of said flexible strip (41) so that the generator lines of the cylindrical shapes of the strip and of the two swellings are substantially parallel to said pivot axis (31),

2. A prosthesis according to claim 1, **characterized by** the fact that each plate is of cylindrical shape, its cross-section constituting the closed curve determining the cylindrical shape and defined perpendicularly to the generator lines, the curve having substantially the shape of a triangle with one of its vertices having an angle of value greater than ninety degrees.

3. A prosthesis according to claim 2, **characterized by** the fact that the slot (46, 47) made in each plate (11, 21) opens out substantially at said one of the vertices of the triangle and is made along the height of the triangle passing via said one vertex.

4. A prosthesis according to claim 3, **characterized by** the fact that the two plates (11, 21) are substantially identical.

5. A prosthesis according to any preceding claim, **characterized by** the fact that it includes at least one piece of reinforcement (A1, A2) embedded in the material in which said flexible hinge (40) is made.

6. A prosthesis according to any preceding claim, **characterized by** the fact that the means (12, 22) for associating one of the plates (11, 21) with a resected end of one of the two bones (01, 02) are constituted by:
• a tab (51, 52);
• means for securing the tab with the plate;
• an optionally self-tapping bone screw (53, 54); and
• means for associating said tab with said bone screw.

7. A prosthesis according to claim 6, **characterized by** the fact that the means for associating said tab with said bone screw are constituted by the fact that the tab (51, 52) is of cylindrical shape and that, in the bone screw, there is made a blind housing (55, 56) of cylindrical shape, the cross-section of said housing being substantially complementary to the cross-section of said tab so that the tab is suitable for sliding in translation in said blind housing.

8. A prosthesis according to claim 7, **characterized by** the fact that the tab (51, 52) is in the form of a cylindrical body of revolution so as to be suitable for swiveling in said blind housing (55, 56) about its longitudinal axis, the two tabs (51, 52) optionally being identical and in the form of cylindrical bodies of revolution.

9. A prosthesis according to any one of claims 6 to 8, **characterized by** the fact that the means for securing the tab with the plate are constituted by one of the following embodiments: making the tab and the plate as a single piece; releasably mounting said tab with said plate.

10. A prosthesis according to any preceding claim, when dependent on claim 2, **characterized by** the fact that said one vertex has an angle of value substantially equal to one hundred and two degrees.

11. A prosthesis according to any one of claims 1 to 9, in combination with an instrument (100) for assisting in assembling said prosthesis, **characterized by** the fact that the instrument comprises:
• a guide (110);
• a passage (120) made through and through in said guide (110) and having a cross-section complementary to the cross-section of said flexible hinge (40), said passage being made in said guide (110) in such a manner as to be accurately in front of said ends of the holes (44, 45) and of the slots (46, 47) situated in the first faces of the two plates (11, 21); and
• a pusher (130) for pushing said flexible hinge (40) in translation in said crosspiece (120) in order to position said flexible hinge (40) in cooperation with the two plates (11, 21) in such a manner that the two swellings (42, 43) and the flexible strip (41) are received respectively in the two holes (44, 45) and in the two slots (46, 47).

12. A prosthesis according to any one of claims 1 to 9, in combination with an instrument (100) according to claim 11, **characterized by** the fact that said guide (110) is constituted by a piece including, at one end (111), an indentation (112) matching at least a portion of the two plates (11, 21) when they are in the position in which they are suitable for being associated by means of the flexible hinge (40).
